# EUROPEAN PATENT APPLICATION

(11) **EP 0 777 406 A1**
(43) Date of publication of application: **04.06.1997**
(21) Application number: 96118591.5
(22) Date of filing: 20.11.1996
(51) Int. Cl.: H05G 1/26, H05G 1/36, H05G 1/46, A61B 6/00

(54) **Automatic exposure metering system for radiology equipment used in mammography**

(30) Priority: 29.11.1995 IT MI952490
(71) Applicant: Gilardoni S.p.A., 20124 Milano (IT)
(72) Inventor: Radogna, Daniele, 22050 Lierna (LC) (IT)
(74) Representative: Petruzziello, Aldo

(57) **Abstract**

An automatic exposure metering system for radiology equipment used in mammography, comprising a short test exposure, in which said test exposure is carried out with a constant radiation dose and radiogenic tube voltage but has a variable duration.

## Description

The present invention relates to a completely automatic exposure metering system for mammography.

In a mammographic examination it is desirable to be able to reduce the examination times to a minimum whilst maximizing the quality of the film obtained through the best contrast possible. These results can be achieved by regulating the voltage supplied to the emission tube and the exposure time according to the characteristics of the breast being examined. Optimal control of both parameters can be achieved manually by operators who are adequately qualified and experienced. The basic parameters for a correct exposure are: the energy of the radiation beam, measured in kV, and the dose, that is the quantity of photons, proportional to the mAs measured; these two parameters can be considered independent for all practical purposes.

The variables that can be controlled by the operator are thus the voltage supplied to the emission tube and the amount of current delivered in the time unit, parameters which must be suitably modulated in order to simplify the examination and obtain a good film, as well as to limit the amount of rays to the minimum adequate dose.

A semi-automatically operating exposure meter, already available for some years and now installed on all mammography equipment, is a device that can reduce the complexity of the examination by providing control of the exposure time once the voltage supplied to the tube has been selected. Said supply voltage is selected by the operator, who establishes it on the basis of an analysis of the type of tissue designed to evaluate its radio-opacity. The analysis is made on the basis of the thickness of the compressed breast subjected to radiological examination, and here what counts is the operator's experience in being able to recognize whether the tissue thickness is due mainly to an adipose component or not. The only information on the radio-opacity of the breast is thus provided by its thickness during compression.

With this type of automatism radiation emission is stopped when the device considers it has reached an optimal dose, calculated by time integrating the current generated in proportional to the rays received by the detection chamber.

Complete automation of the exposure meter on high performance mammographic equipment of modern design provides the additional capability of the system to choose the tube voltage autonomously according to the radio-opacity of the target. This is tested by means of a short test exposure (referred to from now on as preflash) of constant duration, made at a voltage that is normally chosen according to the thickness of the compressed breast. This exposure precedes the emission that is made for diagnostic purposes.

Since the preflash is carried out on the same emulsion on which the diagnostic image will be formed, the completely automatic method must tackle and solve the problems and drawbacks of the present systems.

The main problem of the present systems consists in identifying an adequate preflash voltage, since it is in any case an exposure. In fact, overestimating the voltage leads to overexposure of the film, even for the brief duration of the preflash, because of the rapid increase in the dose depending on the voltage. In this case again, the only available information on which to select the radiogenic tube voltage is the thickness of the compressed breast, a particularly critical choice in the case of a thick breast characterized by a marked presence of adipose tissue, since the selection of a voltage positively correlated to this thickness results in a preflash dose which, being set for a typical opacity, can in itself overexpose the film in the conditions of high transparency of the target associated with a high fat content.

Based on statistics for the USA, the occurrence of such a situation has been estimated to be in the order of at least 5% of all examinations.

In addition, the currently available automatic exposure meters do not guarantee delivery of a correct total dose to the film, since the relationship between dose delivered and density of the film must be modelled taking into account that the preflash delivers the dose in a highly non-linear region of the contrast curve, that the final density on the film is reached by combining two exposures carried out at different voltages and that the functional advantages of fully automatic exposure cannot be achieved at the expense of a significant increase in the time required for calibration of the exposure meter.

The aim of the present invention is to overcome the above mentioned drawbacks and disadvantages, creating a fully automatic exposure metering system that completely removes the causes of error related to the poor correlation between the thickness of the targets and their radio-opacity.

Another aim is to guarantee correct exposure of the plate, without the risk of overexposing it with the preflash.

A further aim is to guarantee easy control of the final density of the film.

Not least of the advantages is the simplicity and speed of the necessary calibration, which requires only minimal operator intervention.

These and other aims are achieved by the device according to the invention, which consists of an automatic exposure meter that carries out a preflash exposure and that is characterised in that said preflash exposure is carried out at a constant dose and voltage and has a variable duration.

Further characteristics of the invention will be made clearer by the detailed description that follows, referring to a purely exemplary and therefore non-limiting embodiment thereof, illustrated in the attached drawings, in which:
Figure 1 is a flow diagram illustrating the constituent parts of a mammography apparatus with automatic exposure according to the invention;
Figure 2 is a diagram that illustrates the relationship existing between preflash exposure time and total dose, for targets of different thicknesses;
Figure 3 is a diagram that illustrates the relationship existing between the duration of the constant-dose preflash and the equivalent thickness of Plexiglas.

With reference to the attached figures, the preflash exposure is characterised by the emission of a constant dose at a constant voltage and by a variable duration ranging from 9 to 100 milliseconds.

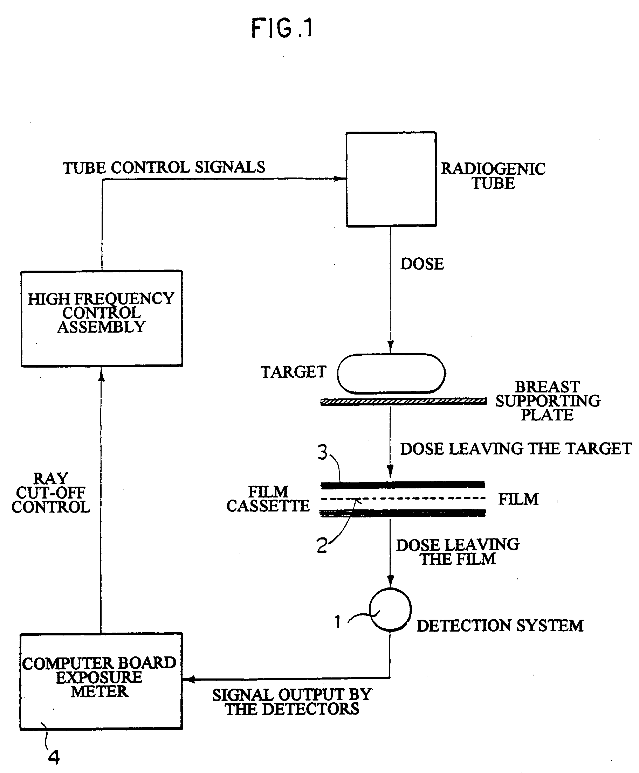

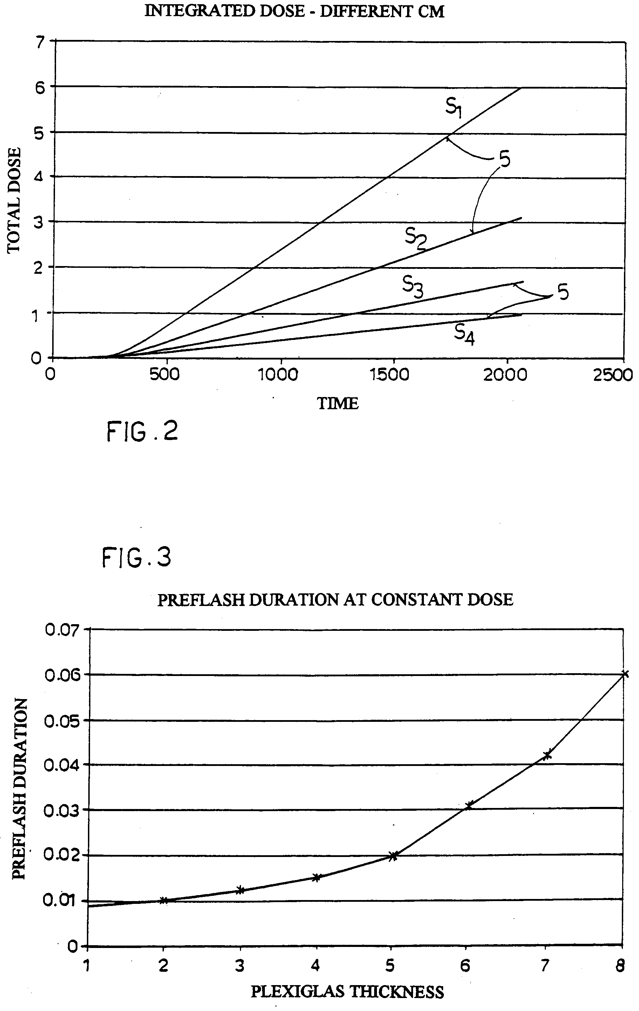

At the start of exposure the rise transient of the dose signal is analysed by measuring the amount of total dose incident on a detection system 1 situated downstream of a film 2 placed inside a cassette 3 (Fig. 1). This dose increases monotonically over time in a typical manner for different thicknesses, as illustrated by the curves 5 in Fig. 2, relating to thicknesses S1-S4 increasing in that order. This dose is instantly compared with a recorded reference dose, the value of which is chosen in such a way that its influence on darkening of the film is negligible.

The constant dose is stored in a computer 4, which carries out the comparison and controls radiation cut-off, through hardware consisting for example of a counter loaded with a certain value which is then decreased down to zero, stopping exposure.

When the total dose value exceeds the predetermined threshold, exposure is stopped and the time that has elapsed since the start of exposure is measured. This time is a function of the radio-opacity of the thickness of material, as can be seen in Figure 3. This opacity is expressed as a so-called equivalent thickness of Plexiglas (ETP), that is to say the thickness of Plexiglas through which the reference dose would have passed in the time measured; with the same radiological parameters.

This equivalence is established by comparison with the data stored during a calibration stage. These data consist of the durations of the exposures necessary to exhaust the predetermined dose with reference thicknesses (for example from 1 to 8 cm in 1-cm steps). Starting from this equivalence it is possible to implement the mechanism of choice of the voltage to be adopted during diagnostic emission in the form of ETP-voltage matching, for example in table form.

If the preflash exposure time were to exceed 100 milliseconds, a safety cut would take place and a maximum voltage, for example, would be chosen for the examination.

The advantages deriving from use of an exposure metering system according to the invention emerge clearly from the above description. Among these are the arbitrary nature of the constant dose, which means that the film is exposed as little as possible during the preflash stage, besides the fact that said constant value, being known in advance, can be subtracted from the final exposure so as to obtain a correctly exposed film with optimal contrast.

## Claims

1. Automatic exposure metering system for radiological equipment used in mammography, comprising a brief test exposure, characterised in that said test exposure is carried out with a constant radiation dose and radiogenic tube voltage and has a variable duration.

2. A system according to claim 1, characterised in that said variable duration preferably ranges between 9 and 100 milliseconds.

3. A system according to any one of the preceding claims, characterised in that the duration of said test exposure is determined on the basis of characteristic curves (5) of the different thicknesses.

4. A system according to any one of the preceding claims, characterized in that said constant dose is chosen in such a way that its effect on blackening of the film (2) is minimal.

5. A system according to any one of the preceding claims, characterised in that it comprises a computer (4) to store a value corresponding to a reference dose and to control exposure cut-off.

6. A system according to any one of the preceding claims, characterised in that the opacity of the irradiated object is established on the basis of an equivalent thickness of Plexiglas calculated by the test exposure.

7. A system according to any one of the preceding claims, characterised in that the choice of voltage supplying the radiogenic tube takes place through a table in which the values of the equivalent thickness of Plexiglas are shown matched to those of the voltage.
